Europäisches Patentamt

European Patent Office

Office européen des brevets

(11) Publication number: **0 398 404 B1**

## EUROPEAN PATENT SPECIFICATION

(45) Date of publication of patent specification: **13.07.94**  (51) Int. Cl.5: **C07C 273/18**, C07C 275/46

(21) Application number: **90200976.0**

(22) Date of filing: **18.04.90**

(54) **A process for the preparation of acyl ureas.**

(30) Priority: **19.04.89 GB 8908868**

(43) Date of publication of application:
**22.11.90 Bulletin 90/47**

(45) Publication of the grant of the patent:
**13.07.94 Bulletin 94/28**

(84) Designated Contracting States:
**BE CH DE DK FR GB IT LI NL SE**

(56) References cited:
**EP-A- 0 225 673**

(73) Proprietor: **SHELL INTERNATIONALE RE-
SEARCH MAATSCHAPPIJ B.V.
Carel van Bylandtlaan 30
NL-2596 HR Den Haag(NL)**

(72) Inventor: **Goodall, Brian Leslie
3959 Clover Hill Road
Akron, Ohio 44313(US)**
Inventor: **Terlouw, Willem
Badhuisweg 3
NL-1031 CM Amsterdam(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to a process for the preparation of certain acyl ureas and to acyl ureas prepared by means of such a process.

Acyl ureas are a known class of commercially available compounds having a wide range of uses. Particular acyl ureas find use as insecticides.

Many processes used hitherto for preparing acyl ureas have relied upon the use of environmentally unacceptable compounds, for example phosgene. A need has arisen, therefore, to develop routes to acyl ureas which do not require the use of such compounds.

European Patent Application publication No. EP 0 225 673 (EP-A-225 673) discloses a process for preparing N-aromatic-N'-acyl ureas comprising reacting an aromatic nitro compound with a primary or secondary amide and carbon monoxide in the presence of a catalyst comprising a Group VIII metal selected from iridium, ruthenium, rhodium, palladium and platinum and a monodentate or bidentate ligand having a lone pair of electrons. Preference is given to the use of palladium in the catalyst, especially in the form of palladium acetate. The catalyst may be supplemented by an acid as a promoter. As an alternative or in addition to the acid, the reaction mixture may also comprise a salt of copper, iron, manganese, chromium, vanadium or cobalt. Preference is given to the use of copper salts. Indeed, the only specific examples of the use of a catalyst comprising a combination of a Group VIII metal and one of the aforementioned metals are Examples 7 and 8 of the specification. Both of these Examples are concerned with a process using a palladium acetate/copper (II) tosylate/2,2'-dipyridyl catalyst, the reaction proceeding at a temperature of 135°C and a pressure of 70 bar.

Surprisingly, it has been found that the use of a catalyst comprising a particular combination of the metals listed in the above mentioned patent specification greatly improves the selectivity of the reaction, increasing the yield of the desired acyl urea.

Accordingly, the present invention provides a process for the preparation of an N-aromatic-N'-acyl urea, which comprises reacting an aromatic nitro compound with a primary or secondary amide and carbon monoxide in the presence of a catalyst comprising palladium, characterised in that the catalyst further comprises a vanadium salt and that the pressure is in the range of from 1 to 10 bar.

Throughout this specification an acyl group should be understood as to be an acyl group derived from an organic carboxylic acid.

The catalyst may comprise palladium present in the form of palladium metal or a palladium compound. If palladium metal is present, it is preferably deposited on an inert carrier, such as carbon or alumina.

Preferably the catalyst comprises a palladium salt or a palladium complex. Suitable palladium salts that may be employed include the palladium halides, expecially palladium chloride, palladium bromide or palladium iodide, palladium carboxylates such as palladium acetate, palladium propionate and palladium isobutyrate.

A catalyst comprising a complex of palladium is especially preferred. Examples of suitable palladium complexes include palladium acetylacetonate, sodium tetrachloropalladate, potassium tetrachloropalladate and potassium tetraiodopalladate. Particularly preferred complexes are those containing carbon monoxide and having the general formula:

$$MPd(CO)X_3$$

wherein M represents a cation which renders the palladium complex soluble in the reaction mixture and X represents a chlorine or bromine atom. Preferably M represents a quaternary onium cation, for example a quaternary ammonium, sulphonium or phosphonium cation. Most preferably M represents a quaternary ammonium cation having the general formula:

$$R^1R^2R^3R^4N$$

wherein each of $R^1$, $R^2$, $R^3$ and $R^4$, which may be the same or different, independently represents a $C_{1-6}$ alkyl group. Preferably each of $R^1$, $R^2$, $R^3$ and $R^4$ represents a $C_{3-5}$ alkyl group, with butyl being especially preferred. X preferably represents a chlorine atom.

The amount of palladium used in the process of the invention is not critical, but is conveniently in the range of from 0.001% w/w to 10% w/w, preferably in the range of from 0.005% w/w to 3% w/w, based upon the amount of the aromatic nitro compound present.

Examples of suitable vanadium salts for inclusion in the catalyst include those having anions selected from halides, in particular chloride, bromide and iodide, sulphate, hydrocarbonylsulphonate, fluoroborate,

2

perchlorate and oxyhalides, in particular oxydichloride, oxydibromide, oxytrichloride and oxytribromide. Preferred salts are the vanadium halides, especially the trihalides, and the vanadium oxyhalides. Vanadium trichloride is especially preferred.

The vanadium salt is preferably present in the form of a solvate selected to be readily soluble in the reaction mixture.

The molar ratio of palladium to vanadium salt is preferably in the range of from 0.01:1 to 10:1.

When palladium is present in the form of a palladium salt or a palladium complex, the catalyst may also comprise an organo-nitrogen compound as a ligand. Examples of organo-nitrogen compounds which may be used include mono-, bi- or poly-cyclic systems containing one or more, especially 1 or 2, nitrogen atoms. Particular examples include pyrrole, pyrolidine, pyridine, piperidine, pyrimidine, 1,10-phenanthroline, pyrazine, benztriazole, morpholine, 2,2'-dipyridyl, 2,2'- biquinoline, bis-pyridylketone, bis-pyridylglyoxal and their alkyl- substituted derivatives. Analogues containing other substituents, for example sulphonyl, carbonyl, alkoxy and halide moieties, may also be use.

Alternatively, non-cyclic amines may also be used, for example tetraethylenediamine, 1,2-bis-(dimethylamino)-ethane, 1,2-bis(diethylamino)-ethane, 1,2-bis(dimethylamino)-propane, 1,2-bis(di-tert-butylamino)-ethane, 1,2-bis(diphenylamino)-prepare and 1,2-bis (diphenylamino)-butane.

When palladium is present in the form of a complex having the general formula given above, the catalyst preferably further comprises a salt having the general formula:

$$M^1X$$

wherein $M^1$ represents a cation which renders the salt soluble in the reaction mixture and X is as hereinbefore defined. $M^1$ preferably represents a quaternary onium cation, for example a quaternary ammonium, sulphonium or phosphonium cation. Most preferably M represents a quaternary ammonium cation having the general formula:

$$R^1R^2R^3R^4N$$

wherein each of $R^1$, $R^2$, $R^3$ and $R^4$, which may be the same or different, independently represents a $C_{1-6}$ alkyl group. Preferably each of $R^1$, $R^2$, $R^3$ and $R^4$ represents a $C_{3-5}$ alkyl group, with butyl being especially preferred. Preferably, M and $M^1$ are the same.

The ligand or the salt of formula $M^1X$ as hereinbefore defined, when present, is preferably present in an amount to give a molar ratio of ligand to palladium in the range of from 0.1:1 to 100:1, especially 0.1:1 to 10:1.

The process of the present invention is suitable for use with aromatic nitro-compounds. The aromatic group in the nitro-compound may be phenyl, naphthyl or heteroaryl, for example pyridyl. The aromatic group may optionally be substituted by one or more inert substituents, such as halogen atoms, alkyl, haloalkyl, alkoxy and haloalkoxy groups. Further subtituents include phenyl, phenoxy, pyridyl and pyridyloxy groups themselves optionally substituted by one or more substituents such as halogen atoms, alkyl, haloalkyl, alkoxy and haloalkoxy groups. Substituent alkyl and alkoxy groups preferably have from 1 to 4 carbon atoms, especially 1 or 2 carbon atoms.

Suitable amides for use in the process of the present invention may be primary or secondary amides. Examples of suitable amides can be represented by the general formula:

$$R^1 - NH - CO - R^2$$

wherein R represents a hydrogen atom or an alkyl or optionally substituted phenyl group, or a group of formula $-COR^3$, wherein $R^3$ represents an alkyl group or an optionally substituted phenyl group, and $R^2$ represents an optionally substituted alkyl, aryl or heteroaryl group.

When $R^1$ or $R^3$ represents an optionally substituted phenyl group, the substituents may be selected from one or more of the inert substituents given above in relation to the aromatic nitro compound. $R^1$ preferably represents a hydrogen atom.

$R^2$ may represent, for example, an alkyl group having from 1 to 10 carbon atoms, preferably from 1 to 6 carbon atoms, optionally substituted by one or more substituents, which may be the same or different and which may, for example, be selected from the group consisting of halogen atoms, alkoxy and haloalkoxy groups, preferably having from 1 to 4 carbon atoms, especially 1 or 2 carbon atoms, and optionally substituted phenyl groups. Alternatively, $R^2$ may represent an optionally substituted phenyl, naphthyl or pyridyl group. Preferably $R^2$ represents an optionally substituted phenyl group. Optional substituents for phenyl, naphthyl or pyridiyl groups may be the same or different and may, for example, be selected from the group consisting of halogen atoms and alkyl, haloalkyl, alkoxy and haloalkoxy groups, and phenyl, phenoxy, pyridyl and pyridyloxy groups themselves optionally substituted by one or more substituents selected from halogen atoms, alkyl, haloalkyl, alkoxy and haloalkoxy groups. Substituent alkyl and alkoxy groups preferably have from 1 to 4 carbon atoms, especially 1 or 2 carbon atoms.

Acyl ureas of particular commercial significance are those having insecticidal activity. Therefore, the process of the present invention finds particular use when the aromatic nitro compound and the amide are selected to yield such compounds. Accordingly, preferred aromatic nitro compounds are nitrobenzenes bearing one or more halogen atoms as substituents. Examples of especially preferred aromatic nitro compounds include 4-chloronitrobenzene, 4-(trifluoromethoxy)nitrobenzene, 2,4-difluoro-3,5-dichloronitrobenzene, 4-(2-chloro-4-trifluoromethylphenoxy)nitrobenzene, 2-fluoro-4-(2-chloro-4-trifluoromethylphenoxy)nitrobenzene and 3,5-dichloro-4-(3-chloro-5-trifluoromethylpyridyl-2-oxy)nitrobenzene.

Although aliphatic amides, for example acetamide, may be used, optionally substituted benzamides are preferred. Especially useful acyl ureas having insecticidal activity can be obtained using benzamide or a halobenzamide bearing one or two halogen atoms, preferably fluorine and/or chlorine atoms. Accordingly, especially preferred benzamides include 2,6-difluorobenzamide, 2,4-difluorobenzamide and 2-chlorobenzamide.

The aromatic nitro compounds and amides useful as starting materials in the process of this invention are either commercially available or can be readily obtained by standard preparative methods.

All of the amide to be reacted may be present with the aromatic nitro compound at the start of the reaction. However, it is preferred that the amide is added continuously or portionwise during the period of the reaction. Continuous addition of the amide is especially preferred.

The preferred rate of supply of the amide to the reaction mixture will depend upon the particular reactants and reaction conditions selected, and is readily determined by routine experimentation.

Preferably the amide is supplied over at least 40%, more preferably at least 50% of the period of the reaction. In certain cases it may be advantageous to supply the amide over at least 80%, and even up to 100% of the period of the reaction.

The amide may be supplied in a molar amount less than the molar amount of the aromatic nitro compound, but preferably in a molar amount at least equal to the molar amount of the aromatic nitro compound.

All of the carbon monoxide to be reacted may be present at the start of the reaction. However, it is preferred that carbon monoxide is passed through the reaction mixture during the period of the reaction.

The process is preferably effected at a temperature in the range of from about 70°C to 160°C, more preferably at a temperature in the range of from 100°C to 140°C, depending upon the stability of the catalyst used.

The process of the present invention may be effected at pressures up to 10 bar. Pressures in the range of from 2 bar to 6 bar are preferred.

If a solvent is employed in the reaction, the solvent may be selected from any non-polar unreactive organic solvent. Examples of suitable solvents include halogenated hydrocarbons, such as chloroform, 1,2-dichloroethane, chlorobenzene and the three dichlorobenzenes; hydrocarbons, such as hexane, cyclohexane, octane, benzene, toluene and the three xylenes; ethers, such as diethyl ether, tetrahydrofuran, dioxane and anisole; and esters, such as ethyl acetate. Preferred solvents are the three dichlorobenzenes, 1,2-dichlorobenzene being especially preferred.

Use of the process of the present invention results in a higher selectivity to the desired product than the processes of the prior art by reducing the level of dehydration of the amide to the corresponding nitrile. The process is suitably carried out in the absence of substantial quantities of hydroxylic compounds, such as water or alcohols, as such compounds yield unwanted by products. The presence of water can lead to formation of the disubstituted urea corresponding to the nitro compound. The amount of water which can be tolerated depends upon the acceptable quantity of the urea present in the final product. The presence of water in the amounts found in technical materials can normally be tolerated. However, any water present may advantageously be removed azeotropically.

Preferably, the reaction mixture contains less than 5% w/w of hydroxylic compounds.

The process of the present invention and its advantages will be further understood from the following Examples, in which each of Examples 1 to 3 relates to a process according to the present invention, whilst Example 4 is a comparative example relating to a process of the prior art. In the following Examples, products were identified by high performance liquid chromatography (HPLC) via comparison with authentic reference samples.

4

Preparation of Catalyst

The palladium complex $(BU_4N)Pd(CO)Cl_3$ was prepared using the method described by Browning et al, J. Chem. Soc. Dalton Trans., (1977), 2061.

The vanadium solvate $VCl_3$. THF was prepared by dissolving vanadium trichloride in boiling tetrahydrofuran. The tetrahydrofuran of the resulting solution was evaporated to yield the required vanadium solvate.

Example 1

Preparation of N'-(4-chlorophenyl)-N-(2,6-difluorobenzoyl)urea

Nitrogen was passed through an autoclave (Hastelloy C - Trade Mark) to render the atmosphere within the autoclave inert. The autoclave was then charged with the solvent 1,2-dichlorobenzene (100 ml), the catalyst components palladium complex $(BU_4N)$ Pd $(CO)Cl_3$ (1.0 mmole, 0.483 g), vanadium solvate $VCl_3$.3THF (3.0 mmoles, 1.12 g), tetra-n-butyl ammonium chloride (2.0 mmoles, 0.55 g), and reactants 4-chloronitrobenzene (50 mmoles, 7.92 g) and 2,6-difluorobenzamide (40 mmoles, 6.28 g). The autoclave was pressurized to 6 bar and the contents heated to a temperature of 130°C with stirring. Carbon monoxide was supplied to the autoclave and bubbled through the contents at a flowrate of 6Nl/h for a period of 21 hours maintaining the temperature of the contents at 130°C. After 21 hours had elapsed, the supply of carbon monoxide was removed, the pressure within the autoclave released and the contents allowed to cool to ambient temperature (20°C). The contents were analysed by HPLC showing a 45% conversion of the 2,6-difluorobenzamide starting material yielding the desired product, N'-(4-chlorophenyl)-N-(2,6-difluorobenzoyl)-urea (17.5 mmoles, 5.43 g) and 2,6-difluorobenzonitrile (0.54 mmoles, 0.075 g). The desired product was recovered as a crystalline solid from the contents of the autoclave by filtration. The selectivity of the reaction to the desired product was 97% and to 2,6-difluorobenzonitrile 3%, based on the conversion of 2,6-difluorobenzamide starting material.

Examples 2 and 3

Preparation of N'-(4-chlorophenyl)-N-(2,6-difluorobenzoyl)urea

The general method of Example 1 was repeated using the same starting materials, namely 4-chloronitrobenzene and 2,6-difluorobenzamide, and similar reaction conditions but using various catalyst components. The details and results of these further preparations are set out in Table 1 below.

Example 4

Preparation of N'-(4-chlorophenyl)-N-(2,6-difluoro-benzoyl)urea

The general method of Example 1 was repeated using the same starting materials and similar reaction conditions, but using as catalyst components Palladium acetate, copper (II) tosylate and 2,2'-dipyridyl, as described and exemplified in EP-A-225 673. The details and results of this preparation are set out in Table 1 below.

The results set out in Table 1 indicate that the use of a catalyst comprising palladium and a vanadium salt in a process according to the present invention results in a significantly greater selectivity to the desired acyl urea product, with a corresponding significant reduction in formation of the corresponding nitrile, compared with the process of the prior art.

TABLE 1

| Example No. | Catalyst Composition (mmoles) | | Reaction Time h. | Pressure bar | Conversion of 2,6-difluoro benzamide % | Selectivity to desired product % | Selectivity to 2,6-difluoro benzonitrile % |
|---|---|---|---|---|---|---|---|
| 1 | $(Bu_4N)Pd(CO)Cl_3$ | (1.0) | 21 | 6 | 45 | 97 | 3 |
| | $VCl_3.3THF$ | (3.0) | | | | | |
| | $Bu_4NCl$ | (2.0) | | | | | |
| 2 | $(Bu_4N)Pd(CO)Cl_3$ | (1.0) | 21 | 6 | 38 | 99 | 1 |
| | $VCl_3.3THF$ | (3.0) | | | | | |
| 3 | $(Bu_4N)Pd(CO)Cl_3$ | (0.33) | 90 | 1 | 45 | 99 | 1 |
| | $VCl_3.3THF$ | (0.93) | | | | | |
| 4 | $Pd(CH_3COO)_2$ | (1.0) | 21 | 6 | 70 | 89 | 11 |
| | $CuTS_2$ | (2.0) | | | | | |
| | 2,2'-dipyridyl | (20) | | | | | |

## Claims

1. A process for the preparation of an N-aromatic-N'-acyl urea, which comprises reacting an aromatic nitro compound with a primary or secondary amide and carbon monoxide in the presence of a catalyst

6

comprising palladium, characterised in that the catalyst further comprises a vanadium salt and that the pressure is in the range of from 1 to 10 bar.

2. A process according to claim 1, characterised in that the vanadium salt is a halide or an oxyhalide.

3. A process according to claim 1 or 2, characterised in that palladium is present in the catalyst in the form of a complex having the general formula:

$$MPd(CO)X_3$$

wherein M represents a cation which renders the palladium complex soluble in the reaction mixture and X represents a chlorine or bromine atom.

4. A process according to claim 3, characterised in that M represents a quaternary ammonium cation having the general formula:

$$R^1R^2R^3R^4N$$

wherein each of $R^1$, $R^2$, $R^3$ and $R^4$, which may be the same or different, independently represents a $C_{1-6}$ alkyl group.

5. A process according to claim 3 or 4, characterised in that the catalyst further comprises a salt having the general formula:

$$M^1X$$

wherein $M^1$ represents a cation which renders the salt soluble in the reaction mixture and X represents a chlorine or bromine atom.

6. A process according to claim 5, characterised in that $M^1$ represents a quaternary ammonium cation having the general formula:

$$R^1R^2R^3R^4N$$

wherein each of $R^1$, $R^2$, $R^3$ and $R^4$, which may be the same or different, independently represents a $C_{1-6}$ alkyl group.

7. A process according to any preceding claim, characterised in that the reaction is effected in the presence of a dichlorobenzene.

8. A process according to any preceding claim, characterised in that the temperature is in the range of from 70 to 160°C.

9. A process according to any preceding claim, characterised in that the amide is supplied continuously or portionwise during the period of the reaction.

10. A process according to claim 9 characterised in that the amide is supplied over at least 50% of the period of the reaction.

11. A process according to any preceding claim, characterised in that the amide is a substituted benzamide.

12. A process according to any preceding claim, characterised in that the aromatic nitro compound is a substituted nitrobenzene.

**Patentansprüche**

1. Ein Verfahren zur Herstellung eines N-aromatischen-N'-Acyl-Harnstoffs, das darin besteht, daß eine aromatische Nitroverbindung mit einem primären oder sekundären Amid und Kohlenmonoxid in Gegenwart eines palladiumhaltigen Katalysators zur Reaktion gebracht wird, dadurch gekennzeichnet, daß der Katalysator außerdem ein Vanadiumsalz enthält und daß der Druck im Bereich zwischen 1 und 10 Bar liegt.

2. Das unter Anspruch Nr. 1 aufgeführte Verfahren, dadurch gekennzeichnet, daß das Vanadiumsalz ein Halogenid oder ein Oxydhalogenid ist.

3. Das unter Anspruch Nr. 1 oder 2 aufgeführte Verfahren, dadurch gekennzeichnet, daß Palladium in der Form eines Komplexes mit der allgemeinen Formel:

$MPd(CO)X_3$

in dem Katalysator enthalten ist, wobei M ein Kation darstellt, durch welches der Palladiumkomplex im Reaktionsgemisch löslich wird, und X ein Chlor- oder Bromatom darstellt.

4. Das unter Anspruch Nr. 3 aufgeführte Verfahren, dadurch gekennzeichnet, daß M ein quaternäres Ammonium-Kation mit der allgemeinen Formel:

$R^1R^2R^3R^4N$

darstellt, wobei $R^1$, $R^2$, $R^3$ und $R^4$, die identisch oder verscheiden sein können, jeweils unabhängig voneinander eine $C_{1-6}$-Alkylgruppe darstellen.

5. Das unter Anspruch Nr. 3 oder 4 aufgeführte Verfahren, dadurch gekennzeichnet, daß der Katalysator außerdem ein Salz mit der allgemeinen Formel:

$M^1X$

darstellt, wobei $M^1$ ein Kation darstellt, durch welches das Salz im Reaktionsgemisch löslich wird, und X ein Chlor- oder Bromatom darstellt.

6. Das unter Anspruch Nr. 5 aufgeführte Verfahren, dadurch gekennzeichnet, daß $M^1$ ein quaternäres Ammonium-Kation mit der allgemeinen Formel:

$R^1R^2R^3R^4N$

darstellt, wobei $R^1$, $R^2$, $R^3$ und $R^4$, die identisch oder verscheiden sein können, jeweils unabhängig voneinander eine $C_{1-6}$-Alkylgruppe darstellen.

7. Das unter einem beliebigen der obenstehenden Ansprüche aufgeführte Verfahren, dadurch gekennzeichnet, daß die Reaktion in Gegenwart eines Dichlorbenzols durchgeführt wird.

8. Das unter einem beliebigen der obenstehenden Ansprüche aufgeführte Verfahren, dadurch gekennzeichnet, daß die Temperatur im Bereich zwischen 70 und 160 °C liegt.

9. Das unter einem beliebigen der obenstehenden Ansprüche aufgeführte Verfahren, dadurch gekennzeichnet, daß das Amid während der Reaktionsdauer kontinuierlich oder portionsweise zugeführt wird.

10. Das unter Anspruch Nr. 9 aufgeführte Verfahren, dadurch gekennzeichnet, daß das Amid während mindestens 50% der Reaktionsdauer zugeführt wird.

11. Das unter einem beliebigen der obenstehenden Ansprüche aufgeführte Verfahren, dadurch gekennzeichnet, daß das Amid ein substituiertes Benzamid ist.

**12.** Das unter einem beliebigen der obenstehenden Ansprüche aufgeführte Verfahren, dadurch gekennzeichnet, daß die aromatische Nitroverbindung ein substituiertes Nitrobenzol ist.

**Revendications**

**1.** Un procédé pour la préparation d'urée acyle N-aromatique-N', lequel suppose que l'on fasse réagir un composé nitro-aromatique avec un amide primaire ou secondaire et un monoxyde de carbone, en présence d' un catalyseur comprenant du palladium, en ceci caractéristique que le catalysateur comprend également du sel de vanadium et que la pression est de l'ordre de 1 a 10 bars.

**2.** Un procédé, conformément à la conclusion 1, en ceci caractéristique que le sel de vanadium est un halite ou un oxyhalite.

**3.** Un procédé, conformément à la conclusion 1 ou 2, en ceci caractéristique que le palladium est présent dans le catalyseur sous la forme d'un complexe correspondant à la formule générale suivante:

MPd (CO) $x_3$

dans laquelle M représente un cation qui rend soluble le complexe de palladium dans le mélange de réaction, et où X représente un atome de chlore ou de brome.

**4.** Un procédé conformément à la conclusion 3, en ceci caractéristique que M représente un cation quaternaire d'ammonium correspondant à la formule générale suivante:

$R^1R^2R^3 R^4 N$

dans laquelle chaque $R^1$, $R^2$, $R^3$ et $R^4$, pouvant être identique ou différent, représente de manière autonome un groupe alcoyle $C_{1-6}$.

**5.** Un procédé conformément à la conclusion 3 ou 4, en ceci caractéristique que le catalyseur comprend également un sel correspondant à la formule générale:

$M^1X$

dans laquelle $M^1$ représente un cation rendant le sel soluble dans le mélange de réaction, et où X représente un atome de chlore ou de brome.

**6.** Un procédé conformément à la conclusion 5, en ceci caractéristique que $M^1$ représente un cation quaternaire d'ammonium correspondant à la formule générale suivante:

$R^1R^2R^3 R^4 N$

dans laquelle chaque $R^1$, $R^2$, $R^3$ et $R^4$, pouvant être identique ou différent, représente de manière autonome un groupe alcoyle $C_{1-6}$.

**7.** Un procédé conformément à n'importe laquelle des conclusions précédentes, en ceci caractéristique que la réaction s'effectue en présence de dichlorobenzène.

**8.** Un procédé conformément à n'importe laquelle des conclusions précédentes, en ceci caractéristique que la température est de l'ordre de 70 à 160°.

**9.** Un procès conformément à n'importe laquelle des conclusions précédentes, en ceci caractéristique que l'amide est fourni de manière continue ou intermittente pendant la période de réaction.

**10.** Un procédé conformément à la conclusion 9, en ceci caractéristique que l'amide est fournie pendant un temps correspondant à au moins 50% de la période de réaction.

11. Un procédé conformément à n'importe laquelle des conclusions précédentes, en ceci caractéristique que l'amide y est un benzamide de substitution

12. Un procédé conformément à n'importe laquelle des conclusions précédentes, en ceci caractéristique que le composé nitro-aromatique y est un nitrobenzène de substitution.